# EUROPÄISCHE PATENTSCHRIFT

(11) **EP 2 904 087 B1**
(45) Veröffentlichungstag und Bekanntmachung des Hinweises auf die Patenterteilung: **24.08.2016**
(21) Anmeldenummer: 13765970.2
(22) Anmeldetag: 29.08.2013
(51) Int. Cl.: C12M 1/06

(54) **RÜHREREINBAUHILFE UND VERFAHREN ZUM EINBAU EINES RÜHRORGANS IN EINEN BIOREAKTOR**
STIRRER INSTALLATION AID AND METHOD FOR INSTALLING A STIRRER ELEMENT IN A BIOREACTOR
SYSTÈME D'AIDE AU MONTAGE D'AGITATEUR ET PROCÉDÉ POUR MONTER UN ORGANE AGITATEUR DANS UN BIORÉACTEUR

(30) Priorität: 01.10.2012 DE 102012019215
(43) Veröffentlichungstag der Anmeldung: 12.08.2015
(73) Patentinhaber: Sartorius Stedim Biotech GmbH, 37079 Göttingen (DE)
(72) Erfinder: DREHER, Thomas, 37083 Göttingen (DE); SCHEIBE, Oliver, 31655 Stadthagen (DE); GRELLER, Gerhard, 37085 Göttingen (DE); HUSEMANN, Ute, 37079 Göttingen (DE); REIF, Oscar-Werner, 30173 Hannover (DE)
(74) Vertreter: Schneider, Peter Christian
(86) Internationale Anmeldenummer: PCT/EP2013/067928
(87) Internationale Veröffentlichungsnummer: WO 2014/053276

(56) Entgegenhaltungen:
- EP-A2- 1 577 376
- EP-B1- 2 024 072
- EP-B1- 2 274 085
- DE-U1-202007 005 868
- DE-U1-202007 007 827

## Beschreibung

### Gebiet der Erfindung

Die Erfindung betrifft die Verwendung einer Hülse, die einen zwischen ihren axialen Enden verlaufenden, von gegenüberliegenden Flanken der Wandung begrenzten Schlitz aufweist, als Rührereinbauhilfe.

Weiterhin betrifft die Erfindung ein Verfahren zum Einbau eines Rührorgans an einer in einem Bioreaktor angeordneten Rührerwelle.

### Stand der Technik

Bei der Inbetriebnahme von Bioreaktoren, die einen Rührer aufweisen, ist es notwendig, an der Rührerwelle des Rührers Rührorgane zu montieren. Um dabei optimale Strömungsverhältnisse gewährleisten zu können, ist darauf zu achten, dass die Rührorgane eine definierte Position an der Rührerwelle haben. Der Einbau sowie das Einstellen der Höhe, d.h. insbesondere der Abstand des Rührorgans zum Reaktorboden und/oder zur Reaktordecke, gestalten sich häufig dabei als komplizierter und zeitaufwendiger Schritt.

Insbesondere bei wieder verwendbaren Bioreaktoren müssen bei der Reinigung die Rührorgane immer wieder entfernt und neu an der Rührerwelle installiert werden. Dies gestaltet sich insofern schwierig, da gleichzeitig der Abstand gemessen und das Rührorgan festgeschraubt werden muss. Zudem ist der Einbauraum häufig relativ schwer zugänglich und die Längenbestimmung und deren Einhaltung während der Montage schwierig.

Aus der DE 20 2007 007 827 U1 ist eine Hülse/Klemmhülse bekannt, die einen zwischen ihren axialen Enden verlaufenden, von gegenüberliegenden Flanken der Wandung begrenzten Schlitz aufweist und aufweitbar ist. Die bekannte Hülse wird in Verbindung mit Klemmfittings für Rohre eingesetzt.

Aus der DE 20 2007 005 868 U1 ist ein Bioreaktor bekannt, in dem mindestens ein Mischer bestehend aus mindestens einem Mischelement und einem am Deckenteil angeordneten Mischerschaft, der von einem außerhalb des Reaktorinnenraumes angeordneten Antrieb antreibbar ist, angeordnet ist. Der Mischerschaft ist aus einer Mehrzahl von ineinander verrastbaren Schaftelementen zusammensetzbar.

Je nach Höhe des verwendeten Reaktorinnenraumes wird der verwendete Mischer bzw. sein Mischerschaft aus zwei oder mehr ineinander verrastbaren Schaftelementen zusammengesetzt.

Weiter ist aus der EP 2 024 072 B1 ein Bioreaktor mit einem Mischerschaft bekannt, an dem zwei Mischorgane voneinander beabstandet angeordnet sind.

### Aufgabenstellung

Aufgabe der vorliegenden Erfindung ist es, die Positionierung von Rührorganen an Rührerwellen zu vereinfachen und die Genauigkeit der Positionierung zu verbessern.

### Darlegung der Erfindung

Die Aufgabe der vorliegenden Erfindung wird durch die Verwendung einer Hülse, die einen zwischen ihren axialen Enden verlaufenden, von gegenüberliegenden Flanken der Wandung begrenzten Schlitz aufweist, als entfernbare Rührereinbauhilfe zum Positionieren eines Rührorgans an einer Rührerwelle eines Bioreaktors gelöst.

Durch die Verwendung einer Rührereinbauhilfe, die als eine auf ihrer Länge geschlitzte Hülse ausgebildet ist, kann diese einfach als eine Art Abstandshalter eingesetzt werden, der nach der Positionierung des Rührorgans und seiner Fixierung leicht seitlich von der Rührerwelle abgezogen werden kann. Dadurch wird die Genauigkeit der Positionierung erheblich verbessert und zugleich wird die Positionierung bzw. die Montage des Rührorgans an der Rührerwelle erheblich vereinfacht.

Gemäß einer bevorzugten Verwendung der Hülse wird die Rührereinbauhilfe auf die Rührerwelle aufgesetzt, wobei sie mit ihrem ersten axialen Ende an die Befestigung der Rührerwelle anschlägt, wobei anschließend das Rührorgan an das freie zweite axiale Ende der auf die Rührerwelle aufgesteckten Rührereinbauhilfe anschlägt und wobei nach der Befestigung des Rührorgans an der Rührerwelle die Rührereinbauhilfe seitlich abgezogen wird. Die Rührereinbauhilfe kann dann bei einer erneuten Montage wieder eingesetzt werden. Die Rührereinbauhilfe wird befestigt, so dass die Rührereinbauhilfe an dem dem Antrieb für das Rührorgan (z. B. einem Motor) zugewandten Ende anschlägt. Dieses Ende kann beispielsweise der Reaktorboden oder eine Anschlagfläche eines Aufnahme- oder Führungsflansches sein.

Gemäß einer bevorzugten Ausführungsform der Erfindung kann die Rührereinbauhilfe aus einem elastischen Material ausgebildet und aufweitbar sein. Durch das elastische Material kann die Rührereinbauhülse mit einer Klemmwirkung auf die Rührerwelle aufgesteckt werden und behält ihre Position bei, was die Handhabung der Rührereinbauhilfe erleichtert. Durch die Aufweitbarkeit der Hülse ist sie zudem problemlos wieder entfernbar.

Gemäß einer bevorzugten Ausführungsform der Erfindung weist die Rührereinbauhilfe einen c-förmigen Querschnitt auf. Grundsätzlich ist es aber auch möglich, die Rührereinbauhilfe beispielsweise mit einem u-förmigen Querschnitt auszubilden.

Die von dem Abstand der axialen Enden bestimmte Hülsenlänge bestimmt ihrerseits die Position des Rührorgans auf der Rührerwelle.

Weiterhin wird das Verfahren zum Einbau eines Rührorgans an einer in einem Bioreaktor angeordneten Rührerwelle in Verbindung mit dem Oberbegriff von Anspruch 7 dadurch gelöst, dass folgende Schritte ausgeführt werden:
a) Aufstecken einer in ihrer axialen Längsrichtung über ihre gesamte Hülsenlänge geschlitzten Hülse, die eine Rührereinbauhilfe bildet, auf die Rührerwelle,
b) Aufstecken des Rührorgans auf das freie Ende der Rührerwelle, bis es an das benachbarte Ende der Hülse anschlägt,
c) Fixieren des Rührorgans an der Rührerwelle, und
d) Entfernen der Rührereinbauhilfe.

Durch das Aufstecken der längsgeschlitzten Hülse, die die Rührereinbauhilfe bildet, auf die Rührerwelle lässt sich das Rührorgan durch Aufsetzen auf das freie Ende der Rührerwelle und Anschlagen an das benachbarte Ende der Hülse einfach und sicher in seine vorgegebene Position bringen und an der Rührerwelle fixieren. Die nicht mehr benötigte Rührereinbauhilfe kann dann leicht entfernt werden. Wegen ihres Längsschlitzes kann die Rührereinbauhilfe einfach seitlich von der Rührerwelle abgezogen werden.

Bei einer weiteren bevorzugten Ausführungsform des erfindungsgemäßen Verfahrens zum Einbau von mehreren Rührorganen an einer in einem Bioreaktor angeordneten Rührerwelle wird nach dem Einbau eines ersten Rührorgans gemäß den Schritten a) bis d) des Anspruchs 7 mindestens ein weiteres Rührorgan an der in dem Bioreaktor angeordneten Rührerwelle eingebaut, wobei folgende Schritte ausgeführt werden:
a') Aufstecken einer in ihrer axialen Längsrichtung über ihre gesamte Hülsenlänge geschlitzten zweiten Hülse, die eine Rührereinbauhilfe bildet, auf das an der Rührerwelle fixierte erste Rührorgan,
b') Aufstecken eines zweiten Rührorgans auf das freie Ende der Rührerwelle, bis es an das benachbarte Ende der zweiten Hülse anschlägt,
c') Fixieren des zweiten Rührorgans an der Rührerwelle,
d') Entfernen der zweiten Hülse und
e) ggf. mehrfaches Wiederholen der Schritte a') bis d'), um weitere Rührorgane auf der Rührerwelle zu fixieren.

Der Vorteil dieser Ausführungsform besteht darin, dass schnell, effizient und in genau reproduzierbaren Abständen entweder mehrere Rührorgane äquidistant an der Rührerwelle fixiert werden können, wenn in den Schritten a') bis d') und ggf. e) jeweils Hülsen gleicher Länge verwendet werden. Bei dieser Variante kann als zweite und weitere Hülse jeweils auch dieselbe Hülse verwendet werden, die bei dem Einbau der vorhergehenden Rührorgane verwendet wurde. Andererseits können für den Einbau mehrerer Rührorgane verschiedene, baugleiche Hülsen verwendet werden. Alternativ können schnell, effizient und in genau reproduzierbaren Abständen Rührorgane in verschiedenen Abständen entlang der Rührerwelle fixiert werden, wenn in den Schritten a') bis d') und ggf. e) jeweils Hülsen verschiedener Längen verwendet werden.

Weitere Merkmale und Vorteile der Erfindung ergeben sich aus der nachfolgenden speziellen Beschreibung und den Zeichnungen.

### Kurzbeschreibung der Zeichnungen

- Figur 1:: eine Seitenansicht teilweise im Schnitt eines Bioreaktors mit einem Rührer, dessen Rührorgan in seiner Position auf der Rührerachse durch eine Einbauhülse bestimmt ist;
- Figur 2:: eine Seitenansicht im Ausriss eines Bioreaktors mit einer Rührerachse;
- Figur 3:: eine Seitenansicht im Ausriss des Bioreaktors von Figur 2 mit einer Rührereinbauhilfe;
- Figur 4:: eine Seitenansicht im Ausriss des Bioreaktors von Figur 3 mit aufgestecktem Rührorgan;
- Figur 5:: eine Seitenansicht im Ausriss des Bioreaktors von Figur 4 mit entfernter Rührereinbauhilfe;
- Figur 6:: eine Seitenansicht einer Rührereinbauhilfe;
- Figur 7:: einen Querschnitt der Rührereinbauhilfe von Figur 6 entlang der Linie VII - VII geschnitten;
- Figur 8:: einen weiteren Querschnitt einer Rührereinbauhilfe entsprechend dem Querschnitt von Figur 7 ebenfalls c-förmig ausgebildet;
- Figur 9:: einen Querschnitt durch eine weitere Rührereinbauhilfe mit u-förmiger Gestaltung;
- Figur 10:: eine Seitenansicht eines weiteren Bioreaktors im Ausriss mit hängend angeordnetem Rührorgan, Rührerwelle und Rührereinbauhilfe und
- Figur 11:: eine Seitenansicht teilweise im Schnitt eines Bioreaktors mit einem Rührer, dessen Rührorgane in ihrer Position auf der Rührerwelle durch Einbauhülsen bestimmt sind.

### Beschreibung bevorzugter Ausführungsformen

Ein Bioreaktor 1 besteht im Wesentlichen aus einem Reaktorinnenraum 2 mit einem Rührer 3.

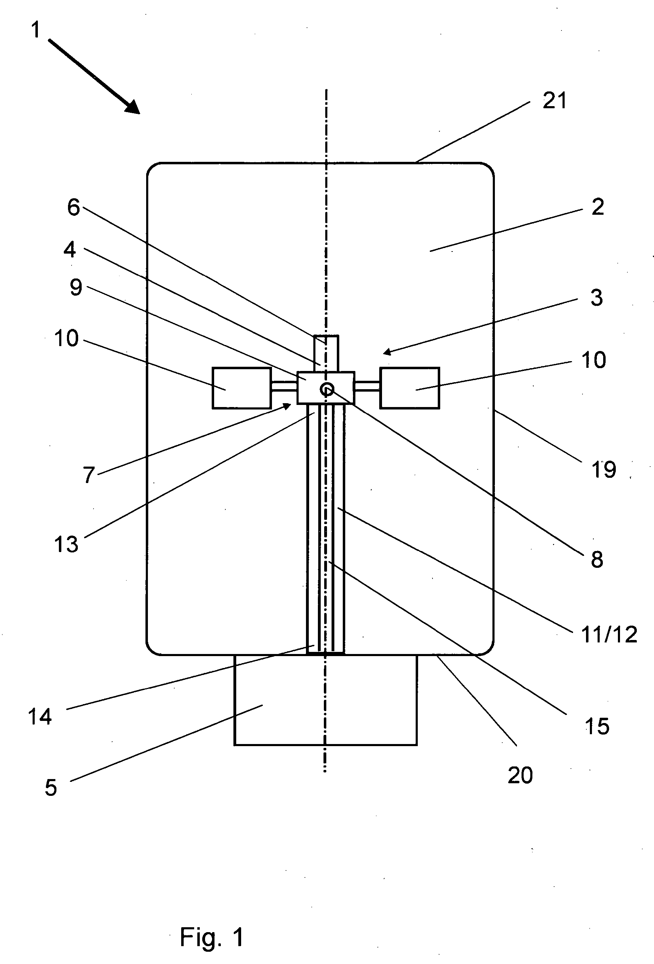

Der Rührer 3 besteht aus einer in den Reaktorinnenraum 2 hineinragenden Rührerwelle 4, die von einem Antrieb 5, z. B. einem Motor, antreibbar ist und an deren dem Antrieb 5 abgewandten freien Ende 6 ein Rührorgan 7 positioniert ist. Im Ausführungsbeispiel der Figur 1 wird das Rührorgan mit einer Schraube 8 an der Rührerwelle 4 fixiert. Das Rührorgan 7 besteht aus einem ringförmigen Verbindungsstück 9, das auf das freie Ende 6 der Rührenrvelle aufsteckbar ist, und radial abstehenden Rührflügeln 10. Auf die Rührerwelle 4 ist eine Rührereinbauhilfe 11 aufgesteckt, die als eine Hülse 12 ausgebildet ist und einen zwischen ihren axialen Enden 13, 14 verlaufenden Schlitz 15 aufweist. Der Schlitz 15 wird von gegenüberliegenden Flanken 16, 17 der Wandung 18 begrenzt.

Die Position des Rührorgans 7 auf der Rührerwelle 4 wird durch die Hülse 12, die als Rührereinbauhilfe 11 verwendet wird und an deren zweites oder oberes axiales Ende 13 bei einem Einbau entsprechend Figur 1 das Rührorgan 7 mit seinem Verbindungsstück 9 anschlägt, vorgegeben. Die Hülse 12 ist elastisch, d.h. federnd ausgebildet, so dass ihr Schlitz 15 so aufweitbar ist, dass sie seitlich auf die Rührerwelle 4 sowohl aufgesteckt als auch von der Rührerwelle 4 abgezogen werden kann.

Der Reaktorinnenraum 2 wird seitlich von einer Reaktorwandung 19, in vertikaler Richtung unten von einem Reaktorboden 20 und in vertikaler Richtung oben von einer Reaktordecke 21 begrenzt.

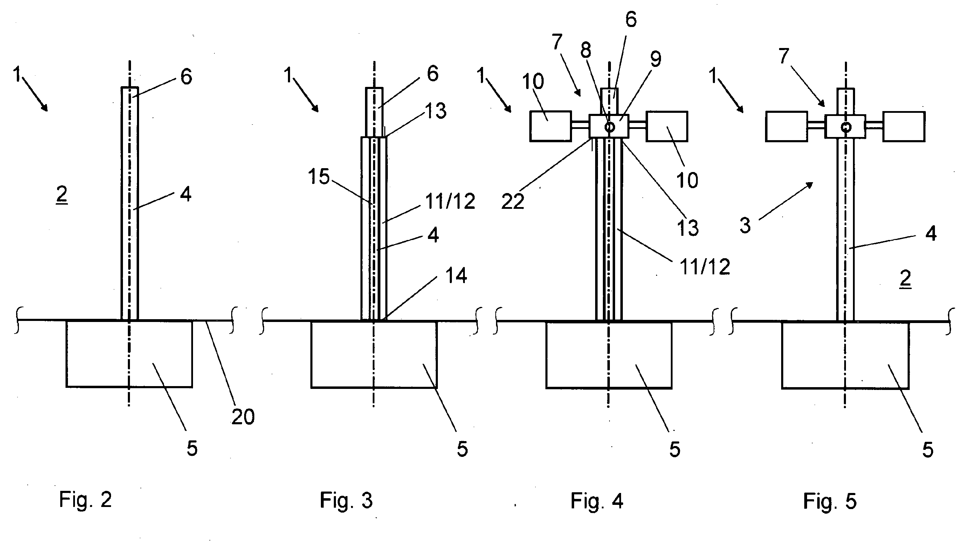

Zur Positionierung des Rührorgans 7 auf der Rührerwelle 4 (siehe Figur 2) wird in einem Schritt a) die Rührereinbauhilfe 11/Hülse 12 auf die Rührerwelle 4 aufgesteckt, wobei sie mit ihrem unteren oder ersten axialen Ende 14 beispielsweise an den benachbarten Reaktorboden 20 (siehe Figur 3) anschlägt. In einem Folgeschritt b) wird das Rührorgan 7 mit seinem Verbindungsstück 9 auf das freie Ende 6 der Rührerwelle so aufgesetzt, dass es mit seiner Anschlagfläche 22 an dem oberen oder zweiten axialen Ende 13 der Hülse 12 anschlägt. In einem Schritt c) wird das Rührorgan 7 durch die Schraube 8 an der Rührerwelle 4 fixiert (siehe Figur 4). In einem Folgeschritt d) wird die Rührereinbauhilfe 11/Hülse 12 dann durch seitliches Abziehen von der Rührerwelle 4 entfernt.

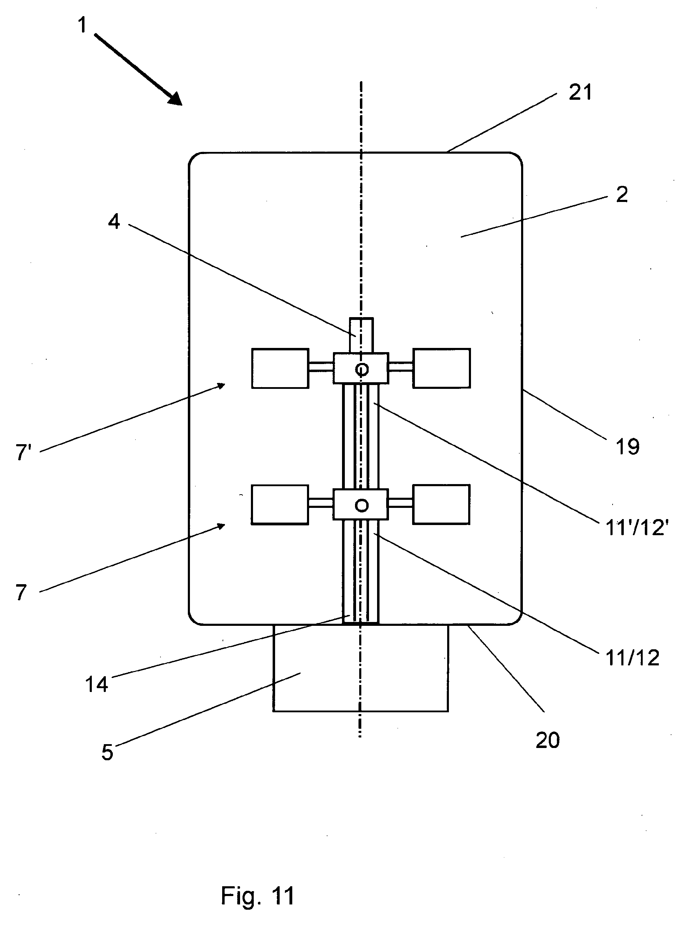

Zum Einbau von mehreren Rührorganen 7, 7' an der in dem Bioreaktor 1 angeordneten Rührerwelle 4 (siehe Figur 11) wird nach dem Einbau des ersten Rührorgans 7 mindestens ein weiteres Rührorgan 7' an der in dem Bioreaktor 1 angeordneten Rührerwelle 4 eingebaut, wobei folgende Schritte ausgeführt werden:
a') Aufstecken einer in ihrer axialen Längsrichtung über ihre gesamte Hülsenlänge geschlitzten zweiten Hülse 12', die ebenfalls eine Rührereinbauhilfe 11' bildet, auf das an der Rührerwelle 4 fixierte erste Rührorgan 7,
b') Aufstecken eines zweiten Rührorgans 7' auf das freie Ende der Rührerwelle 4, bis es an das benachbarte Ende der zweiten Hülse 12' anschlägt,
c') Fixieren des zweiten Rührorgans 7' an der Rührerwelle 4,
d') Entfernen der zweiten Hülse 12'.

Gegebenenfalls erfolgt in einem Schritt e) ein mehrfaches Wiederholen der Schritte a') bis d'), um weitere Rührorgane 7, 7' auf der Rührerwelle 4 zu fixieren.

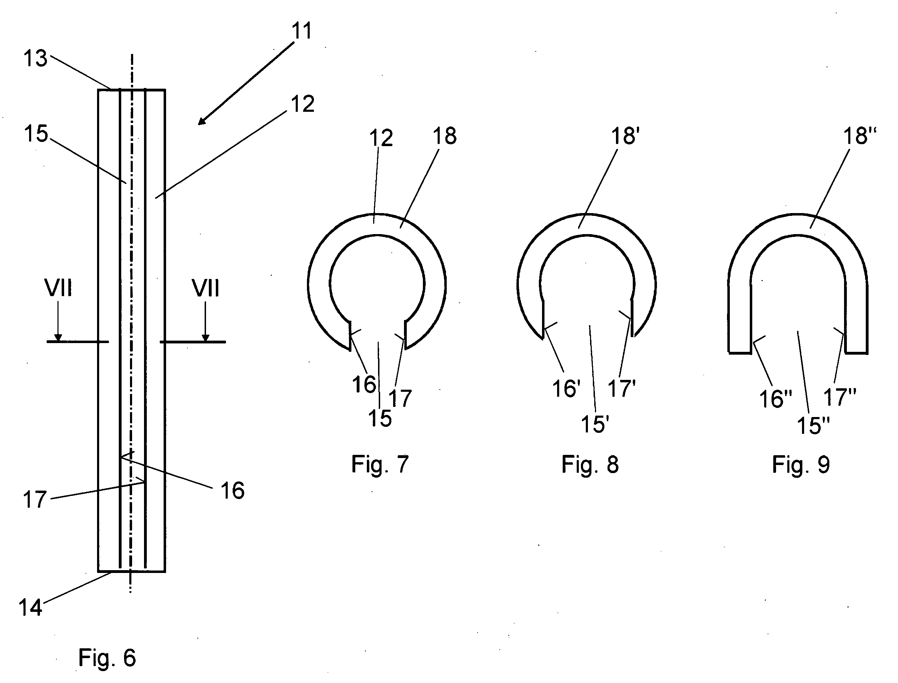

Der Querschnitt der Hülse 12 kann entsprechend den Figuren 7 und 8 c-förmig ausgebildet sein. Die Breite des Schlitzes 15, 15', die durch den Abstand der einander zugewandten Flanken 16, 17 bzw. 16', 17' gebildet wird, ist insbesondere von der Elastizität des Hülsenmaterials abhängig. Grundsätzlich kann der Querschnitt der Rührereinbauhilfe 11 aber auch u-förmig ausgebildet sein, wobei der Abstand der gegenüberliegenden Flanken 16", 17" des Schlitzes 15" etwa dem Innendurchmesser der Hülse 12 entspricht.

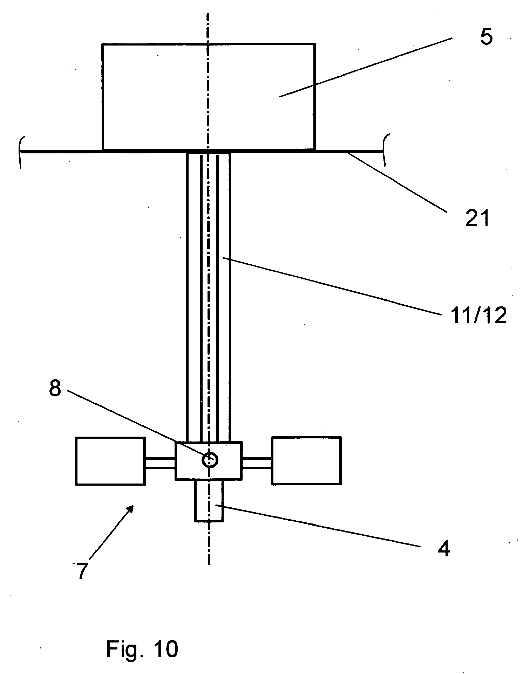

Während entsprechend den Figuren 1 bis 9 und 11 die Rührerwelle 4 stehend angeordnet ist, kann sie auch entsprechend der Figur 10 hängend angeordnet sein.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum an Variationsmöglichkeiten an die Hand gegeben. Insbesondere können das Rührorgan 7, die Rührerwelle 4 und die Rührereinbauhilfe 11 auch hängend am Bioreaktor 1 oder an der Reaktorwandung 19 seitlich angeordnet sein. Die Rührereinbauhilfe 11 soll zumindest so gestaltet sein, dass sie die Rührerwelle 4 mindestens teilweise umschließt und festklemmbar sowie seitlich entfernbar ist.

### Bezugszeichenliste

- 1: Bioreaktor
- 2: Reaktorinnenraum
- 3: Rührer
- 4: Rührerwelle von 3
- 5: Antrieb
- 6: freies Ende von 4
- 7, 7': Rührorgan
- 8: Schraube
- 9: Verbindungsstück von 7
- 10: Rührflügel von 7
- 11: Rührereinbauhilfe
- 12, 12': Hülse
- 13: zweites axiales Ende von 12
- 14: erstes axiales Ende von 12
- 15, 15', 15": Schlitz von 12
- 16, 16', 16": Flanke von 12
- 17, 17', 17": Flanke von 12
- 18, 18', 18": Wandung
- 19: Reaktorwandung von 1
- 20: Reaktorboden von 1
- 21: Reaktordecke von 1
- 22: Anschlagfläche von 9

## Patentansprüche

1. Verwendung einer Hülse (12), die einen zwischen ihren axialen Enden (13, 14) verlaufenden, von gegenüberliegenden Flanken (16, 16', 16", 17, 17', 17") der Wandung (18, 18', 18") begrenzten Schlitz (15, 15', 15") aufweist, als entfernbare Rührereinbauhilfe (11) zum Positionieren eines Rührorgans (7) an einer Rührerwelle (4) eines Bioreaktors (1).

2. Verwendung nach Anspruch 1,
**dadurch gekennzeichnet,**
**dass** die Rührereinbauhilfe (11) auf die Rührerwelle (4) aufgesetzt wird und mit dem ersten axialen Ende (14) an die Befestigung der Rührerwelle (4) anschlägt, dass das Rührorgan (7) auf die Rührerwelle (4) mit der Rührereinbauhilfe (11) aufgesteckt und an das freie zweite axiale Ende (13) der Rührereinbauhilfe (11) angeschlagen wird, und
**dass** nach der Befestigung des Rührorgans (7) an der Rührerwelle (4) die Rührereinbauhilfe (11) seitlich abgezogen wird.

3. Verwendung nach Anspruch 1 oder 2,
**dadurch gekennzeichnet,**
**dass** die Rührereinbauhilfe (11) aus einem elastischen Material ausgebildet und aufweitbar ist.

4. Verwendung nach einem der Ansprüche 1 bis 3,
**dadurch gekennzeichnet,**
**dass** die Rührereinbauhilfe (11) einen c-förmigen Querschnitt aufweist.

5. Verwendung nach einem der Ansprüche 1 bis 3,
**dadurch gekennzeichnet,**
**dass** die Rührereinbauhilfe (11) einen u-förmigen Querschnitt aufweist.

6. Verwendung nach einem der Ansprüche 1 bis 5,
**dadurch gekennzeichnet,**
**dass** die von dem Abstand der axialen Enden (13, 14) bestimmte Hülsenlänge die Position des Rührorgans (7) auf der Rührerwelle (4) bestimmt.

7. Verfahren zum Einbau eines Rührorgans (7) an einer in einem Bioreaktor (1) angeordneten Rührerwelle (4),
**dadurch gekennzeichnet,**
**dass** folgende Schritte durchgeführt werden:
a) Aufstecken einer in ihrer axialen Längsrichtung über ihre gesamte Hülsenlänge geschlitzten Hülse (12), die eine Rührereinbauhilfe (11) bildet, auf die Rührerwelle (4),
b) Aufstecken des Rührorgans (7) auf das freie Ende (6) der Rührerwelle (4), bis es an das benachbarte Ende der Hülse (12) aufschlägt,
c) Fixieren des Rührorgans (7) an der Rührerwelle (4), und
d) Entfernen der Rührereinbauhilfe (11).

8. Verfahren nach Anspruch 7,
**dadurch gekennzeichnet,**
**dass** die Rührereinbauhilfe (11) im Schritt d) seitlich von der Rührerwelle (4) abgezogen wird.

9. Verfahren nach einem der Ansprüche 7 oder 8,
**dadurch gekennzeichnet,**
**dass** zum Einbau von mehreren Rührorganen (7', 7") an einer in einem Bioreaktor (1) angeordneten Rührerwelle (4) nach dem Einbau eines ersten Rührorgans (7) mindestens ein weiteres Rührorgan (7', 7") an der in dem Bioreaktor (1) angeordneten Rührerwelle (4) eingebaut wird, wobei folgende Schritte ausgeführt werden:
a') Aufstecken einer in ihrer axialen Längsrichtung über ihre gesamte Hülsenlänge geschlitzten zweiten Hülse (12'), die eine Rührereinbauhilfe (11') bildet, auf das an der Rührerwelle (4) fixierte erste Rührorgan (7),
b') Aufstecken eines zweiten Rührorgans (7') auf das freie Ende der Rührerwelle (4), bis es an das benachbarte Ende der zweiten Hülse (12') anschlägt, c') Fixieren des zweiten Rührorgans (7') an der Rührerwelle (4),
d') Entfernen der zweiten Hülse (12') und
e) ggf. mehrfaches Wiederholen der Schritte a') bis d'), um weitere Rührorgane (7") auf der Rührerwelle (4) zu fixieren.

## Claims

1. Use of a sleeve (12), which comprises a slot (15, 15', 15") extending between the axial ends (13, 14) of the sleeve and delimited by opposite flanks (16, 16', 16", 17, 17', 17") of the wall (18, 18', 18"), as a removable stirrer installation aid (11) for positioning a stirrer element (7) on a stirrer shaft (4) of a bioreactor (1).

2. The use according to Claim 1,
**characterised in that**
the stirrer installation aid (11) is push-fitted onto the stirrer shaft (4), the first axial end (14) abutting on the mounting of the stirrer shaft (4); that the stirrer element (7) is push-fitted onto the stirrer shaft (4) with the stirrer installation aid (11), abutting against the free, second axial end (13) of the stirrer installation aid (11), and
**in that** the stirrer installation aid (11) is removed laterally after the stirrer element (7) has been fastened to the stirrer shaft (4).

3. The use according to Claim 1 or 2,
**characterised in that**
the stirrer installation aid (11) is made of an elastic material and is expandable.

4. The use according to any of Claims 1 to 3,
**characterised in that**
the stirrer installation aid (11) has a "C"-shaped cross section.

5. The use according to any of Claims 1 to 3,
**characterised in that**
the stirrer installation aid (11) has a "U"-shaped cross section.

6. The use according to any of Claims 1 to 5,
**characterised in that**
the sleeve length determined by the spacing of the axial ends (13, 14) determines the position of the stirrer element (7) on the stirrer shaft (4).

7. A method for installing a stirrer element (7) on a stirrer shaft (4) arranged in a bioreactor (1),
**characterised in that**
the following steps are carried out:
a) push-fitting onto the stirrer shaft (4) a sleeve (12) which is slotted in the axial longitudinal direction over the entire length of the sleeve, which sleeve forms a stirrer installation aid (11),
b) push-fitting the stirrer element (7) onto the free end (6) of the stirrer shaft (4) until it abuts against the adjacent end of the sleeve (12),
c) fixing the stirrer element (7) on the stirrer shaft (4), and
c) removing the stirrer installation aid (11).

8. The method according to Claim 7,
**characterised in that**
the stirrer installation aid (11) is pulled off the stirrer shaft (4) laterally in step d).

9. The method according to any of Claims 7 or 8,
**characterised in that**
to install multiple stirrer elements (7', 7") on a stirrer shaft (4) arranged within a bioreactor (1) following installation of a first stirrer element (7), at least one additional stirrer element (7',7") is installed on the stirrer shaft (4) arranged within the bioreactor (1), with the following steps being carried out:
a) push-fitting a second sleeve (12') which is slotted in the axial longitudinal direction over the entire length of the sleeve, which sleeve forms a stirrer installation aid (11'), onto the first stirrer element (7) fixed to the stirrer shaft (4),
b) push-fitting a second stirrer element (7') onto the free end of the stirrer shaft (4) until it abuts against the adjacent end of the second sleeve (12'),
c) fixing the second stirrer element (7') on the stirrer shaft (4),
d) removing the second sleeve (12'), and
e) if necessary repeating steps a') to d') multiple times in order to fix additional stirrer elements (7") to the stirrer shaft (4).

## Revendications

1. Utilisation d'une douille (12) comprenant une fente (15, 15', 15") s'étendant entre les extrémités axiales (13, 14) et limitée par des flancs opposés (16, 16', 16", 17, 17', 17") de la paroi (18, 18', 18"), en tant que système amovible d'aide au montage d'agitateur (11) pour le positionnement d'un élément agitateur (7) sur un arbre d'agitateur (4) d'un bioréacteur (1).

2. Utilisation selon la revendication 1,
**caractérisée en ce que**
le système d'aide au montage d'agitateur (11) est emmanché sur l'arbre d'agitateur (4), la première extrémité axiale (14) butant contre le montage de l'arbre d'agitateur (4) ; que l'élément agitateur (7) est emmanché sur l'arbre d'agitateur (4) avec le système d'aide au montage d'agitateur (11), butant contre la seconde extrémité axiale libre (13) du système d'aide au montage d'agitateur (11), et
**en ce que** le système d'aide au montage d'agitateur (11) est enlevé latéralement après la fixation de l'élément agitateur (7) à l'arbre d'agitateur (4).

3. Utilisation selon la revendication 1 ou 2,
**caractérisée en ce que**
le système d'aide au montage d'agitateur (11) est en matériau plastique expansible.

4. Utilisation selon l'une quelconque des revendications 1 à 3,
**caractérisée en ce que**
le système d'aide au montage d'agitateur (11) est profilé en C.

5. Utilisation selon l'une quelconque des revendications 1 à 3,
**caractérisée en ce que**
le système d'aide au montage d'agitateur (11) est profilé en U.

6. Utilisation selon l'une quelconque des revendications 1 à 5,
**caractérisée en ce que**
la longueur de douille déterminée par l'espacement des extrémités axiales (13, 14) détermine la position de l'élément agitateur (7) sur l'arbre d'agitateur (4).

7. Procédé de montage d'un élément agitateur (7) sur un arbre d'agitateur (4) disposé dans un bioréacteur (1),
**caractérisé en ce que**
ledit procédé consiste à :
a) emmancher sur l'arbre d'agitateur (4) une douille (12) fendue sur toute sa longueur dans sa direction axiale, qui forme un système d'aide au montage (11),
b) emmancher l'élément agitateur (7) sur l'extrémité libre (6) de l'arbre d'agitateur (4) jusqu'à ce qu'il bute contre l'extrémité voisine de la douille (12),
c) fixer l'élément agitateur (7) sur l'arbre d'agitateur (4), et
d) enlever le système d'aide au montage (11).

8. Procédé selon la revendication 7,
**caractérisé en ce que**
le système d'aide au montage d'agitateur (11) est enlevé latéralement de l'arbre d'agitateur (4) à l'étape d).

9. Procédé selon l'une quelconque des revendications 7 ou 8,
**caractérisé en ce que**
pour monter plusieurs éléments agitateurs (7', 7") sur un arbre d'agitateur (4) disposé dans un bioréacteur (1) après le montage d'un premier élément agitateur (7), au moins un élément agitateur supplémentaire (7',7") est monté sur l'arbre d'agitateur (4) disposé dans le bioréacteur (1), ledit procédé consiste à :
a) emmancher sur le premier élément agitateur (7) fixé sur l'arbre d'agitateur (4) une seconde douille (12') fendue sur toute sa longueur dans sa direction axiale, qui sert de système d'aide au montage (11'),
b) emmancher un second élément agitateur (7') sur l'extrémité libre de l'arbre d'agitateur (4) jusqu'à ce qu'il bute contre l'extrémité voisine de la seconde douille (12'),
c) fixer le second élément agitateur (7') sur l'arbre d'agitateur (4),
d) enlever la seconde douille (12'), et
e) si nécessaire, répéter les étapes a') à d') plusieurs fois pour fixer les éléments agitateurs supplémentaires (7") à l'arbre d'agitateur (4).
